(19) Europäisches Patentamt

European Patent Office

Office européen des brevets

(11) **EP 0 822 803 B1**

(12) **EUROPÄISCHE PATENTSCHRIFT**

(45) Veröffentlichungstag und Bekanntmachung des Hinweises auf die Patenterteilung:
**08.01.2003 Patentblatt 2003/02**

(21) Anmeldenummer: **97906806.1**

(22) Anmeldetag: **20.02.1997**

(51) Int Cl.$^7$: **A61K 7/06**, A61K 7/48

(86) Internationale Anmeldenummer:
**PCT/EP97/00797**

(87) Internationale Veröffentlichungsnummer:
**WO 97/030680 (28.08.1997 Gazette 1997/37)**

(54) **KOSMETISCHES MITTEL MIT EINEM GEHALT AN ILEXHARZ, VERFAHREN ZUR GEWINNUNG VON ILEXHARZ UND DURCH DIESES VERFAHREN ERHÄLTLICHES ILEXHARZ**

COSMETIC AGENT WITH AN ILEX RESIN CONTENT, PROCESS FOR EXTRACTION OF ILEX RESIN AND ILEX RESIN OBTAINED THEREBY

AGENT COSMETIQUE CONTENANT DE LA RESINE D'ILEX, PROCEDE POUR L'EXTRACTION DE CETTE RESINE D'ILEX ET RESINE D'ILEX AINSI OBTENUE

(84) Benannte Vertragsstaaten:
**DE ES FR GB IT**

(30) Priorität: **22.02.1996 DE 19606545**

(43) Veröffentlichungstag der Anmeldung:
**11.02.1998 Patentblatt 1998/07**

(73) Patentinhaber: **Wella Aktiengesellschaft**
**64274 Darmstadt (DE)**

(72) Erfinder:
- **KRIPP, Thomas**
  **D-64407 Fränkisch-Crumbach (DE)**
- **BORMUTH, Hiltrud**
  **D-69488 Birkenau (DE)**
- **FRANZKE, Michael**
  **D-64380 Rossdorf (DE)**
- **BAECKER, Sabine**
  **D-65428 Rüsselsheim (DE)**
- **KISCHKA, Karl-Heinz**
  **D-64293 Darmstadt (DE)**
- **SCHRÖDER, Friedel**
  **D-64293 Darmstadt (DE)**

(56) Entgegenhaltungen:
EP-A- 0 519 777          WO-A-89/00042
FR-A- 2 584 726          FR-A- 2 609 395

- DATABASE WPI Section Ch, Week 9513 Derwent Publications Ltd., London, GB; Class A96, AN 95-093751 XP002030490 & JP 07 017 847 A (SANSEI SEIYAKU KK), 20.Januar 1995
- DATABASE WPI Section Ch, Week 9629 Derwent Publications Ltd., London, GB; Class D21, AN 96-283731 XP002030491 & JP 08 120 255 A (HASEGAWA CO LTD), 14.Mai 1996
- DATABASE WPI Section Ch, Week 9252 Derwent Publications Ltd., London, GB; Class D22, AN 92-424610 XP002030492 & BR 9 101 754 A (DIAURUS MINERACAO LTDA), 24.November 1992
- P.H.LIST & L. HÖRHAMMER: "HAGERS HANDBUCH DER PHARMAZEUTISCHEN PRAXIS, BAND 5: CHEMIKALIEN UND DROGEN (H-M)." 1976, SPRINGER-VERLAG, BERLIN XP002030489 siehe Seite 224, Zeile 7 - Seite 226, Zeile 49

## Beschreibung

Die Erfindung betrifft ein kosmetisches Mittel mit einem Gehalt an Ilexharz, welches aus Blättern von Ilex aquifolium oder Ilex paraguariensis isoliert wird, ein Verfahren zur Gewinnung dieses Ilexharzes sowie das gemäß diesem Verfahren gewonnene Ilexharz.

Gutes Aussehen wird heute weitgehend als unverzichtbarer Bestandteil der Lebensqualität angesehen. Eine besondere Rolle spielen dabei die Haut und die Frisur. Zum Verändern und Gestalten der Frisur werden die unterschiedlichsten Mittel und Hilfsmittel eingesetzt.

Schönes Haar wird in der Regel mit gesundem Haar gleichgesetzt. Der Schönheitsbegriff definiert sich aus einer Reihe von Faktoren, zu denen ganz wesentlich die Begriffe "Glanz" und "Farbtiefe" gehören.

Der Glanz ist physikalisch definiert als der Quotient aus dem gerichtet und dem diffus reflektierten Anteil des auf eine Fläche auffallenden Lichtstromes. Somit ist der Glanz eines Gegenstandes eng korreliert mit dessen Oberflächenbeschaffenheit, genauer gesagt mit dessen Rauhigkeit. Mit steigender Rauhigkeit steigt auch der Anteil an diffus reflektiertem Licht und sinkt somit der Glanz.

Als "Farbtiefe" oder "Farbsättigung" wird das Verhältnis der reinen Farbe zum Weißanteil definiert. Der Weißanteil setzt sich (bei additiver Farbenmischung) zusammen aus den drei Grundfarben "rot", "blau" und "grün". Bei gegebenem Farbton einer Oberfläche entscheidet zusätzlich deren Rauhigkeit über die Farbtiefe; je glatter eine Oberfläche ist, um so geringer ist der unspezifisch zurückgestreute Weißanteil des eingestrahlten Lichtes und um so "satter" erscheint die Farbe. Daher wirken nasse (oder polierte) Gegenstände im allgemeinen farbintensiver als trockene.

Zur Erzielung von Glanz und Farbtiefe ist es also erforderlich, die betreffende Oberfläche zu glätten. Dies kann unter anderem dadurch erreicht werden, daß ein dünner, durchsichtiger Belag mit möglichst hohem Brechungsindex aufgebracht wird, der feine Unebenheiten der Oberfläche ausgleicht.

Für Haare kann dieser Oberflächenbelag - in geeigneten Rezepturen appliziert - aus verschiedenen Substanzklassen aufgebaut werden. Zu diesem Zweck wurden in der Vergangenheit vor allem Mineralöle aus verschiedenen Fraktionen, Vaseline, diverse Polymere sowie in neuerer Zeit in verstärktem Maße Silikonöle in Haarbehandlungsmitteln verwendet. Der Nachteil dieser Stoffe liegt in einer schlechten biologischen Abbaubarkeit, zuweilen fettigem Aussehen der Haare sowie gelegentlich auftretenden physiologischen Verträglichkeitsproblemen.

Aufgabe der Erfindung war es daher, ein kosmetisches Mittel zur Verfügung zu stellen, mit welchem sich eine Glättung der Haaroberfläche und eine Verstärkung von Glanz und Farbtiefe des Haares erzielen läßt und welches die genannten Nachteile nicht aufweist.

Neben Glanz und Farbtiefe zählen zu den erwünschten Eigenschaften des Haares insbesondere auch noch Elastizität, Frisierbarkeit, Stabilität und Volumen der Frisur. Die Haare werden durch Einwirkungen verschiedener Art in ihren physikalischen, chemischen und morphologischen Eigenschaften meist negativ beeinflußt. So wird das Haar durch kosmetische Behandlungen wie wiederholtes Bleichen, Dauerwellen und Färben, aber auch schon durch häufiges Waschen mit entfettenden Tensiden, durch Klimaeinflüsse wie Luftfeuchte und Temperaturunterschiede oder durch die intensive Einwirkung von Sonnenlicht sowie durch mechanische Behandlung wie Bürsten, Kämmen, Frottieren, besonders im Bereich der Haarspitzen, stark strapaziert und geschädigt. Das Haar wird spröde und verliert seinen Glanz. Das derart geschädigte Haar lädt sich beim Bürsten und Kämmen elektrostatisch auf. Die Haaroberfläche wird aufgerauht, und es kommt zu Verfilzungen und Verknotungen. Die Folge ist eine außerordentlich schlechte Kämm- und Entwirrbarkeit der Haare.

Haarpflege- und Haarreinigungsmittel mit einer kämmbarkeitsverbessernden und pflegenden Wirkung, welche eine Verbesserung des Haarzustandes bewirken, haben daher eine erhebliche Bedeutung. In Haarpflege- und Reinigungsmitteln werden zu diesem Zwecke üblicherweise kationische Tenside, kationische Polymere oder Silikonverbindungen eingesetzt. Sie bewirken eine gute Kämmbarkeit, Entwirrung und guten Griff der Haare im feuchten Zustand, vermindern aber bei zufriedenstellenden Naßkriterien eine gute Frisurenerstellung, Elastizität, Stabilität, Halt und Volumen der Frisur im trockenen Zustand.

Eine weitere Aufgabe der Erfindung war es daher, ein kosmetisches Mittel zur Verbesserung von Elastizität, Frisierbarkeit, Stabilität und Volumen der Frisur zur Verfügung zu stellen

Neben den Mitteln zum Pflegen und Reinigen der Haare und den Mitteln zur Farbveränderung und Farbvertiefung spielen Mittel zur Haarverformung eine besondere Rolle. Man unterscheidet hier zwischen den Mitteln zur permanenten Haarverformung, den Dauerwellmitteln und Mitteln zur temporären Haarverformung, den Stylingprodukten.

Haarstylingprodukte oder Haarfestigungsmittel können in unterschiedlichster Form (Lotion, Schaum, Spray, Gel, Creme) appliziert werden. Basis aller Haarfestigungsmittel sind Polymere natürlichen oder synthetischen Ursprungs, die den wesentlichen Beitrag zur Haarfestigung liefern. Da die heute vorhandenen Polymere allein nicht in der Lage sind, alle Anforderungen an ein Haarfestigungsmittel zu erfüllen, ist es notwendig, neben diesen Polymeren Zusatzstoffe, die eine Verbesserung bestimmter Eigenschaften bedingen, einzusetzen.

Zu den Anforderungen an ein Haarfestigungsmittel gehört, daß das Mittel einen guten Halt der Frisur gewährleisten soll, verbunden mit einem angenehmen Griff, einer guten Kämmbarkeit und einem guten Glanz. Gleichzeitig sollen die Haare möglichst unbelastet wirken und eine gute Elastizität haben. Da Zusatzmittel, die die Elastizität des Haares verbessern, recht selten sind, war es Aufgabe dieser Erfindung, ein kosmetisches Mittel zur Verfügung zu stellen, das die Elastizität von Stylingprodukten verbessert.

Die menschliche Haut ist zahlreichen schädlichen Einflüssen ausgesetzt. So wird sie durch Umwelteinflüsse wie z. B. Trockenheit und Kälte strapaziert. Insbesondere die Haut von Hausfrauen, Ärzten und Friseuren, aber auch von zahlreichen anderen Berufsgruppen, wird durch wiederholtes Waschen mit netzenden oder extrahierenden Stoffen oder durch den Kontakt mit Chemikalien geschädigt. Dazu gehört auch die Auslaugung durch Wasser, aggressive Chemikalien wie beispielsweise Chlorwasser, Salzwasser und saures Regenwasser, UV-Licht, oxidativer Streß und Austrocknung durch Hitze, beispielsweise hervorgerufen durch die Sonne oder einen Haarfön.

Um die Wirkung derartiger Belastungen zu verringern, können Stoffe auf die Haut aufgebracht werden, die einen Schutzfilm bilden. Sie sollten hydrophob sein, da die schädlichen Einflüsse bevorzugt in Verbindung mit Wasser auftreten - andererseits sollten sie jedoch mittels Seife wieder auswaschbar sein.

Aufgabe kosmetischer Mittel zur Hautbehandlung ist es, die Haut vor den geschilderten schädlichen Umwelteinflüssen zu schützen, den Verlust an natürlichem Hauttalg und Feuchtigkeit zu ersetzen sowie im Falle einer eingetretenen Schädigung die Wiederherstellung der Hautfunktionen zu unterstützen.

Sämtliche Aufgaben werden gelöst durch den Einsatz von einem Harz, welches aus den Blättern von Ilex aquifolium (Stechpalme) oder Ilex paraguariensis (Matestrauch) isoliert wird.

Die vorliegende Erfindung betrifft den natürlichen Oberflächenbelag der Blätter von Stechpalmen (Ilex aquifolium) und des Matestrauches (Ilex paraguariensis).

Die Blätter der Stechpalme sind von sattgrünem, ledrig glänzendem Aussehen und beachtlicher Stabilität. In einigen Ländern werden sie als Zimmerschmuck zur Weihnachtsdekoration eingesetzt. Dabei überstehen sie viele Wochen in geheizter, trockener Zimmerluft, ohne daß ihr attraktives Aussehen nennenswert Schaden nimmt. Offenbar verfügen diese Blätter über einen sehr wirkungsvollen Schutzmantel, der ihnen nicht nur die tiefgründige Farbe und den prächtigen Glanz verleiht, sondern sie auch unter erschwerten Bedingungen vor Austrocknung und unkontrolliert eindringendem Luftsauerstoff schützt; es kann Jahre dauern, bis ein abgerissenes Ilexblatt Welkungserscheinungen zeigt und das ansonsten sehr empfindliche Chlorophyll verblaßt oder die glatte Oberfläche faltig wird.

Überraschend wurde gefunden, daß sich dieser Schutzmantel isolieren und die Wirkung auf die Belange der Kosmetik übertragen läßt.

Durch Extraktion von Ilexblättern mit geeigneten organischen Lösungsmitteln und anschließender Entfernung des Chlorophylls kann ein Rohstoff erhalten werden, der hellgelb, hydrophob und von harzartiger Konsistenz ist.

Es zeigte sich, daß sich nicht nur aus den Blättern der Stechpalme, sondern auch aus den Blättern des verwandten Matestrauches ein gleichwertiges Oberflächenmaterial mit sehr ähnlichen Eigenschaften gewinnen läßt.

Gegenstand der vorliegenden Erfindung ist daher ein kosmetisches Mittel mit einem Gehalt an Ilexharz, welches aus Blättern von Ilex aquifolium oder Ilex paraguariensis isoliert wird.

In einer weiteren Ausführungsform enthält das erfindungsgemäße kosmetische Mittel zur Haarbehandlung neben dem Ilexharz zusätzlich mindestens ein natürliches oder synthetisches Polymer.

Die natürlichen und synthetischen Polymere sind bevorzugt ausgewählt aus den Gruppen der festigenden und der verdickenden Polymere. Die Polymere können in Mengen von 0,1 bis 20 Gewichtsprozent eingesetzt werden und in gelöster Form oder als Dispersion verwendet werden.

Eine bevorzugte Ausführungsform ist ein kosmetisches Mittel zur Festigung der Haare, welches dadurch gekennzeichnet ist, daß es

(A) 0,001 bis 10 Gew. %, vorzugsweise von 0,01 bis 3,5 Gew % Ilexharz

(B) 0,01 bis 25 Gew. %, vorzugsweise 0,1 bis 20 Gew. % eines natürlichen oder synthetischen Polymeren

(C) 30 bis zu 99,89 Gew. % eines geeigneten organischen, wäßrig/organischen oder wäßrigen Lösungsmittelsystems

sowie gegebenenfalls optionale Zusätze zur weiteren Verbesserung des Produktprofils enthält.

Ein weiterer Gegenstand der Erfindung ist ein kosmetisches Mittel zur Hautbehandlung mit einem Gehalt an Ilexharz, welches aus Blättern von Ilex aquifolium oder Ilex paraguariensis isoliert wird. Für das erfindungsgemäße Hautbehandlungsmittel konnte gegenüber einem Standardpräparat ein stabilerer Schutzfilm nachgewiesen werden.

Weiterhin bewirkt ein teilweiser Ersatz von Paraffinen durch Ilexharz in Emulsionen eine Verbesserung der Lagerstabilität bei erhöhten Temperaturen.

Ein weiterer Gegenstand der Erfindung ist ein Verfahren zur Gewinnung von Ilexharz aus Blättern von Ilex aquifolium oder Ilex paraguariensis durch Lösungsmittelextraktion in an sich bekannter Weise mit semipolaren oder apolaren, wasserfreien organischen Lösungsmitteln oder mit überkritischem Kohlendioxid. In einer bevorzugten Ausführungsform werden

(a) die Blätter mindestens zweimal mit Essigsäureethylester heiß extrahiert und heiß filtriert und das Filtrat zur Trockne eingeengt, anschließend wird

(b) der Rückstand in einem unpolaren Lösungsmittel aufgenommen, in der Wärme Bleicherde zugegeben, zum Sieden erhitzt, heiß filtriert und das Filtrat zur Trockne eingeengt.

Die Extraktionen des Verfahrensschrittes (a) können zur Ausbeuteverbesserung mehrmals wiederholt werden. Auch eine kontinuierliche Extraktion ist geeignet. Bei kontinuierlicher Extraktion muß das Medium mindestens 60° Celsius am Extraktionsgut aufweisen. Geeignete Lösungsmittel für den Extraktionsschritt (a) sind destillierbare, möglichst wasserfreie organische Lösungsmittel mit mindestens 2 Kohlenstoffatomen oder überkritisches $CO_2$. Bevorzugt sind semipolare bis apolare Lösungsmittel. Beim Reinigungsschritt (b) werden Chlorophyll, Triterpene, Coffein und Glykoside entfernt.

Das Ilexharz stellt chemisch ein Gemisch aus freien Fettsäuren, Kohlenwasserstoffen und Fettsäureestern dar, ist von orange-gelber, durchscheinender Farbe und zäher, harziger Konsistenz.

Wird das erfindungsgemäße kosmetische Mittel zur Haarbehandlung eingesetzt, so enthält es zwischen 0,001 und 10 Gewichtsprozent Ilexharz, vorzugsweise zwischen 0,1 und 5 Gewichtsprozent. Dieses Mittel kann sowohl ausgespült werden als auch im Haar verbleiben.

Das erfindungsgemäße kosmetische Mittel weist, wenn es zur Haarbehandlung eingesetzt wird, einen pH-Wert zwischen 2 und 8, vorzugsweise zwischen 4 und 7 auf.

Das kosmetische Mittel kann für die Verwendung zur Haarbehandlung gemäß der vorliegenden Erfindung zusätzlich alle diejenigen Bestandteile enthalten, die in Haarbehandlungsmitteln üblicherweise eingesetzt werden, insbesondere anionische, kationische, amphotere oder nichcionische Tenside, Emulgatoren, Schaumsynergisten; Schaumstabilisatoren; Sequestriermittel, Naturstoffe; Pigmente; Parfümöle in einer Menge von 0,1 bis 5,0 Gewichtsprozent; Trübungsmittel, wie zum Beispiel Ethylenglykoldistearat, in einer Menge von etwa 0,5 bis 5,0 Gewichtsprozent; Perlglanzmittel, wie zum Beispiel ein Gemisch aus Fettsäuremonoalkylolamid und Ethylenglykoldistearat, in einer Menge von etwa 1,0 bis 10,0 Gewichtsprozent; Verdickungsmittel, wie beispielsweise Kokosfettsäurediethanolamid oder Hydroxyalkylcellulose, in einer Menge von 0,5 bis 10,0 Gewichtsprozent; physiologisch verträgliche organische oder anorganische Säuren und Puffersubstanzen, wie beispielsweise Zitronensäure, Weinsäure, Milchsäure, Ameisensäure, Glyoxylsäure, Natriumcitrat oder Natriumphosphat in einer Menge von 0,1 bis 1,0 Gewichtsprozent; sowie Haar- und Produktfärbestoffe, wie zum Beispiel Fluorescein-Natriumsalz, Gelb ZN3 (C. I. 47055), in einer Menge von 0,1 bis 1,0 Gewichtsprozent; weiterhin haarpflegende Zusätze wie zum Beispiel Aminosäuren, Proteine, Kräuterextrakte, Betaine, Vitamine, Kohlenhydrate und -derivate, Harnstoff, ätherische Öle, Fettsäureester, Fettalkohole, Fettsäureglyceride, ethoxylierte oder propoxylierte gesättigte Fettalkohole; natürliche, modifizierte natürliche oder synthetische Polymere, wie zum Beispiel Schellack, kationische, anionische oder nichtionische Cellulosederivate, Chitosan, kationische Chitin- oder Chitosanderivate oder Polymersiate von Acrylsäurederivaten; Pflegestoffe, wie zum Beispiel Lanolinderivate, Cholesterin und Pantothensäure, in einer Menge von 0,1 bis 10 Gewichtsprozent; außerdem physiologisch verträgliche anorganische Salze, wie zum Beispiel Natriumchlorid und Natriumsulfat; sowie ferner Feuchthaltemittel; Lichtschutzmittel; Antioxidantien; Komplexbildner; Antischuppenwirkstoffe; pflegende Lipide mineralischen oder biogenen Ursprungs, wie zum Beispiel kosmetische Öle, Fette, Terpene, Steroide und Wachse sowie Konservierungsstoffe, soweit solche Zusätze nützlich und zweckmäßig erscheinen und mit den Bestandteilen des erfindungsgemäßen Mittels verträglich sind.

Das erfindungsgemäße kosmetische Mittel zur Haarbehandlung weist bevorzugt einen Gehalt an Ilexharz, Tensiden, Emulgatoren und organischen oder anorganischen Säuren oder Puffersubstanzen auf.

Wird das erfindungsgemäße kosmetische Mittel als Hautoder Haarreinigungsmittel verwendet, so enthält es anionische, amphotere oder nichtionische Tenside in einer Menge von 0,01 bis 40 Gew. %, vorzugsweise 0,1 bis 25 Gew. %, Wasser in einer Menge von 50 bis 99 Gew. % und der pH-Wert liegt zwischen 3 und 8, vorzugsweise zwischen 4 und 7. Die genannten Tenside können auch in Gemischen eingesetzt werden.

Als anionische Tenside können z. B. Acylglutamate, Sarcosinate, Acylpeptide, Salze von Carbonsäuren, Taurate, Carbonsäureester und deren Salze, Carbonsäureether und deren Salze, Phosphorsäureester und deren Salze, Sulfonsäuren und deren Salze, Alkylethersulfate, Alkylsulfate, Isethionate eingesetzt werden, wobei Alkali- oder Erdalkalisalze von Laurylethersulfaten besonders bevorzugt sind.

Als amphotere Tenside können z. B. N-Alkylbetaine, N-Alkylaminobetaine, Fettsäureamidoalkylbetaine, Fettsäureamidoalkylsulfobetaine, Carboxylderivate von Imidazol eingesetzt werden. Bevorzugt wird Kokosfettsäureamidopropylbetain eingesetzt, das beispielsweise von der Fa. Goldschmidt unter der Handelsbezeichnung Tego® Betain L5045 vertrieben wird.

Als nichtionische Tenside können z. B. Alkylpolyglucoside, Alkanolamide, Monoglyceride, ethoxylierte Alkohole, Polyglycerinfettsäureester eingesetzt werden. Bevorzugt wird Alkylpolyglucosid, wie es z. B. von der Firma Henkel unter der Handelsbezeichnung Plantaren® 2000 CS/UP oder Plantaren® 818 UP oder von der Firma Seppic unter der Handelsbezeichnung Oramix® NS 10 vertrieben wird, eingesetzt.

Eine besonders bevorzugte Ausführung kann kationische Polymere in einer Einsatzmenge von 0,01 - 8 Gew. %, bevorzugt von 0,05 bis 5 Gew. %, enthalten, z. B. ein mit Dimethylsulfat quaternisiertes Vinylpyrrolidon/Dimethylaminoethylmethacrylat-Copolymer, das beispielsweise von der Fa. GAF unter der Handelsbezeichnung Gafquat® 755 N vertrieben wird, oder kationische Celluslose, wie sie unter der Bezeichnung Ucare® Polymer JR von der Fa. Union Carbide vertrieben wird.

Wird das erfindungsgemäße kosmetische Mittel zur Haarpflege eingesetzt und als Emulsion oder Dispersion konfektioniert, so enthält es zusätzlich zum Ilexharz Lipide, wie z. B. Fettalkohole, Paraffinöle, Fettsäureglycerinester, Silikonverbindungen oder höhere Kohlenwasserstoffe in einer Menge von 0,01 - 30 Gew. % sowie Emulgatoren. Als Lipid werden geradkettige Fettalkohole mit n Kohlenstoffatomen in einer Menge von 0,05 - 18 Gew. %, wobei n die Zahl 8 - 22 darstellt, oder ein Gemisch solcher Fettalkohole, wobei n die durchschnittliche Anzahl der Kohlenstoffatome angibt, besonders bevorzugt. Geeignete Fettalkohole/Fettalkoholgemische werden z. B. von der Fa. Henkel unter der Handelsbezeichnung Lanette® 14, 16, 18, 22 und Lanette® 0 vertrieben.

Als höherer Kohlenwasserstoff wird bevorzugt Isododecan in einer Einsatzmenge von 0,05 bis 15 Gew. % verwendet.

Als Emulgatoren werden kationische, anionische, amphotere oder nichtionische Tenside, bevorzugt in einer Menge von 0,03 bis 8 Gew. %, eingesetzt. Beispiele für geeignete kationische Tenside als Emulgator sind Alkyldimethylbenzylammoniumchloride oder -bromide, Cetyltrimethylammoniumchlorid oder -bromid, Tetradecyltrimethylammoniumchlorid oder -bromid, Dialkyldimethylammoniumchloride oder -bromide, Alkylpyridiniumsalze wie z. B. Lauryl- oder Cetylpyridiniumchlorid, Alkylamidoethyltrimethylammoniumethosulfate, Esterquats wie Dipalmitoylethyldimethylammoniumchlorid, das beispielsweise von der Firma Akzo unter der Handelsbezeichnung Armosoft® VGH-70 vertrieben wird sowie Verbindungen mit kationischem Charakter wie Aminoxide, beispielsweise Alkylmethylaminoxide oder Alkylaminoethyldimethylaminoxide.

Die Mittel enthalten als kationische Tenside bevorzugt Cetyltrimethylammoniumchlorid und/oder Distearyldimethylammoniumchlorid in einer Einsatzmenge von 0,03 bis 8 Gew. %.

Beispiele für geeignete anionische Tenside als Emulgator sind Acylglutamate, Sarcosinate, Acylpeptide, Salze von Carbonsäuren, Tauraten, Carbonsäureester und deren Salze, Carbonsäureether und deren Salze, Phosphorsäureester und deren Salze, Sulfonsäuren und deren Salze, Alkylethersulfate, Alkylsulfate, Isethionate. Die Mittel enthalten als anionisches Tensid bevorzugt Natriumcetylstearylsulfat in einer Einsatzmenge von 0,03 bis 8 Gew. %.

Beispiele für geeignete amphotere Tenside als Emulgator oder Coemulgator sind N-Alkylbetaine, N-Alkylaminobetaine, Fettsäureamidoalkylbetaine, Fettsäureamidoalkylsulfobetaine, Carboxylderivate von Imidazol, Capryloiminodipropionat, das beispielsweise von der Firma Akzo unter dem Handelsnamen Ampholak® YJH-40 vertrieben wird. Die Mittel enthalten als amphotere Tenside bevorzugt Cocoamidopropylbetain und/oder Stearyldimethylglycin in einer Einsatzmenge von 0,03 bis 8 Gew. %. Beispiele für geeignete nichtionische Tenside als Emulgator oder Coemulgator sind Alkylpolyglucoside, Alkanolamide, Monolgyceride, ethoxylierte Alkohole, Polyglycerinfettsäureester, Lecithin, Cholesterin. Die Mittel enthalten bevorzugt Alkylpolyglucoside, die z. B. von der Firma Henkel unter der Handelsbezeichnung Plantaren® 1200 CS/UP, Plantaren® 2000 CS/UP oder Plantaren® 818 UP vertrieben werden, in einer Einsatzmenge von 0,03 bis 8 Gew. %.

Das erfindungsgemäße kosmetische Mittel zur Haarpflege weist bevorzugt einen Wassergehalt von 75 bis 99 Gew. % auf, kann Alkohol in einer Menge von 0,1 bis 25 Gew. % enthalten und weist einen pH-Wert von 2 bis 8, bevorzugt von 2,5 bis 7 auf. Als Alkohole kommen hierbei insbesondere die für kosmetische Zwecke üblicherweise verwendeten niederen Alkohole mit 2 bis 4 Kohlenstoffatomen wie z. B. Ethanol oder Isopropanol in Betracht.

Wird das erfindungsgemäße kosmetische Mittel zur Festigung der Haare eingesetzt, so liegt es vorzugsweise in Form einer alkoholischen, wäßrigen oder wäßrig-alkoholischen Lösung vor. Als geeignete organische Lösungsmittel sind geradkettige oder verzweigte Alkohole mit einer Kettenlänge von $C_1$ bis $C_5$ mit bis zu 3 Hydroxylgruppen zu nennen. Weiterhin geeignet sind geradkettige oder verzweigte Kohlenwasserstoffe mit einer Kettenlänge von $C_1$ bis $C_8$. Als weiteres Lösungsmittel neben diesen sind Glykolether und Wasser zu nennen. Als bevorzugte Lösungsmittel sind Alkohole mit einer Kettenlänge bis $C_4$, Kohlenwasserstoffe mit einer Kettenlänge bis $C_7$ sowie Wasser in Kombination mit geeigneten Lösungsvermittlern zu nennen. Die Konzentration dieser Lösungsmittel kann zwischen 30 und 99,89 Gew. % betragen.

Als optionale Bestandteile können für das erfindungsgemäße kosmetische Mittel zur Festigung der Haare folgende Stoffklassen genannt werden:

Anionische, nichtionische, kationische und amphotere Tenside und Emulgatoren; flüchtige und nichtflüchtige Silikonverbindungen, Lösungsvermittler, Entschäumer, Kämmbarkeitsverbesserer, Parfümöle, Pflanzenextrakte, Proteine und Proteinhydrolysate, native Öle, höhere Kohlenwasserstoffe, wie z. B. Paraffine, Neutralisationsmittel, Konservierungsmittel, UV-Absorber, Antioxidantien, Farbstoffe, Pigmente sowie Treibgase.

Das erfindungsgemäße kosmetische Mittel zur Festigung oder zur Pflege der Haare kann unter Verwendung eines Treibmittels oder mit Hilfe einer mechanisch betriebenen Sprühvorrichtung versprüht werden oder mit Hilfe einer Schaumerzeugungsvorrichtung als Schaum abgegeben werden.

Wenn das erfindungsgemäße kosmetische Mittel mit Hilfe eines Treibmittels versprüht wird, so enthält es bevorzugt 3 bis 75 Gewichtsprozent des Treibmittels und wird in einem Druckbehälter abgefüllt.

Als Treibmittel sind beispielsweise niedere Alkane, wie zum Beispiel n-Butan, i-Butan und Propan oder deren Gemische oder auch Dimethylether und Fluorkohlenwasserstoffe wie beispielsweise F 152 (1,1-Difluorethan)oder F 134 (Tetrafluorethan) sowie ferner bei den in Betracht kommenden Drücken gasförmig vorliegende Treibmittel, wie beispielsweise $N_2$, $N_2O$ und $CO_2$ sowie Gemische der vorstehend genannten Treibmittel geeignet.

Unter mechanischen Sprühvorrichtungen oder Schaumerzeugungsvorrichtungen sind solche Vorrichtungen zu verstehen, welche das Versprühen oder Aufschäumen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in den das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden. Als geeignete mechanische Schaumerzeugungsvorrichtung kann beispielsweise der in der EP-B 0 0460 154 beschriebene Aufsatz mit einer Schaumerzeugungseinrichtung auf einem flexiblen Behälter verwendet werden.

Wird das erfindungsgemäße kosmetische Mittel zur Haarpflege eingesetzt, so wird es folgendermaßen angewandt:

Nach der Haarwäsche werden in dem handtuchtrockenen Haar je nach Haarfülle 5 bis 30 g des Mittels verteilt und ca. 3 - 15 Minuten einwirken gelassen. Anschließend wird das Mittel ausgespült, das Haar durchgekämmt, gegebenenfalls zur Firsur geformt und getrocknet. Eine bevorzugte Ausführung des erfindungsgemäßen Mittels erlaubt den Verbleib im Haar d. h. es wird nicht ausgespült und erspart dem Anwender dadurch einen Arbeitsgang.

Wird das erfindungsgemäße kosmetische Mittel zur Hautbehandlung eingesetzt, so enthält es zwischen 0,001 und 50 Gewichtsprozent Ilexharz, vorzugsweise zwischen 0,1 und 10 Gewichtsprozent. Es kann in Form von Lösungen, Suspensionen, Pasten, Gelen oder Emulsionen, vorzugsweise jedoch in Form von Öl-in-Wasser- oder Wasser-in-Öl-Emulsionen, vorliegen und vorzugsweise als Gesichtswasser, Hautcreme, Körperlotion, Gesichtspackung oder Gesichtsmaske konfektioniert sein.

Das erfindungsgemäße kosmetische Mittel zur Hautbehandlung kann zusätzlich alle diejenigen Bestandteile enthalten, die in Hautbehandlungsmittel üblicherweise eingesetzt werden, insbesondere anionische, nichtionische, kationische, amphotere oder zwitterionische Tenside, beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Cetyl- oder Stearylalkohol, Alkylbetaine, Alkylaminobetaine, Alkylsulfobetaine und Fettsäurealkylamidobetaine in einer Menge von 0,01 bis 5,0 Gewichtsprozent; Sequestriermittel; Emulgatoren; Naturstoffe, wie zum Beispiel Vitamine, vorzugsweise die Vitamine F und B6, D-Panthenol, Aminosäuren wie Betain, Cystein, Alanin, Valin oder Tyrosin, Proteine, Kohlenhydrate und -derivate oder Pflanzenextrakte, Pigmente, Parfümöle, ätherische Öle, in einer Menge von 0,5 bis 5,0 Gewichtsprozent; Trübungsmittel, wie zum Beispiel Ethylenglykoldistearat, in einer Menge von etwa 0,5 bis 5,0 Gewichtsprozent; Feuchthaltemittel, wie beispielsweise Glycerin, Polyole, Hyaluronsäure und Harnstoff, in einer Menge von 0,05 bis 20 Gewichtsprozent, vorzugsweise 0,1 bis 10 Gewichtsprozent; Perlglanzmittel, wie zum Beispiel ein Gemisch aus Fettsäuremonoalkylolamid und Ethylenglykoldistearat, in einer Menge von etwa 1,0 bis 10,0 Gewichtsprozent; Verdikkungsmittel, wie beispielsweise Kokosfettsäurediethanolamid oder Hydroxyalkylcellulose, in einer Menge von 0,1 bis 1,0 Gewichtsprozent; organische oder anorganische Säuren oder Puffersubstanzen, wie beispielsweise Natriumcitrat oder Natriumphosphat, in einer Menge von 0,1 bis 1,0 Gewichtsprozent; sowie Farbstoffe wie zum Beispiel Fluorescein-Natriumsalz, Gelb ZN3 (C. I. 47 055), in einer Menge von 0,1 bis 1,0 Gewichtsprozent; weiterhin hautpflegende Zusätze, wie zum Beispiel pflegende Lipide mineralischen oder biogenen Ursprungs, Fettsäureester, Fettalkohole, Fettsäureglyceride, Terpene, Steroide; natürliche, modifizierte natürliche oder synthetische Polymere, wie zum Beispiel kationische, anionische oder nichtionische Cellulosederivate, Chitosan, kationische Chitin- oder Chitosanderivate, Pflegestoffe, wie zum Beispiel Lanolinderivate, Cholesterin, Allantoin, Alpha-Bisabolol, Azulen und Pantothensäure, in einer Menge von 0,1 bis 10 Gewichtsprozent; außerdem physiologisch verträgliche anorganische Salze, wie zum Beispiel Natriumchlorid und Natriumsulfat; sowie ferner Lichtschutzmittel; Antioxidantien; Komplexbildner; Titandioxid; kosmetische Öle und Wachse sowie Konservierungsstoffe, soweit solche Zusätze nützlich und zweckmäßig erscheinen und mit den Bestandteilen der verwendeten Zubereitung verträglich sind.

Der pH-Wert des erfindungsgemäßen kosmetischen Mittels zur Hautbehandlung beträgt vorzugsweise 4 bis 7 und kann mit physiologisch verträglichen organischen oder anorganischen Säuren oder Basen, beispielsweise mit Benzoesäure, Zitronensäure, Ameisensäure oder Essigsäure, Sorbinsäure, Natriumhydroxid, Ammoniak oder Mono- oder Triethanolamin eingestellt werden. Das erfindungsgemäße kosmetische Mittel zur Hautbehandlung kann wasserfrei sein oder bis zu 99,5 Gewichtsprozent Wasser enthalten. Bevorzugt weist das erfindungsgemäße kosmetische Mittel zur Hautbehandlung einen Wassergehalt von 50 bis 80 Gewichtsprozent auf.

Als chemisch unveränderter Naturscoff ist Ilexharz trotz seiner hohen Hydrophobizität biologisch abbaubar, als Bestandteil eines weitverbreiteten Nahrungsmittels und aufgrund der chemischen Analyse kann es als toxikologisch unbedenklich eingestuft werden.

In einem Haarglanzpräparat führt der Einsatz von Ilexharz zu einer Farbintensivierung gegenüber dem mit einer Vergleichsrezeptur behandelten Haar. Zusätzlich hinterläßt das Ilexharzprodukt einen stärkeren Glanz.

Des weiteren weisen die mit dem erfindungsgemäßen Haarpflege- und/oder Haarreinigungsmittel behandelten Haare eine sehr gute Naßkämmbarkeit und einen angenehmen Griff auf. Das Haar hat einen ansprechenden Glanz, besitzt Elastizität und Spannkraft. Die Frisur läßt sich leicht erstellen und zeichnet sich durch guten Halt und Volumen aus.

Haare, die mit dem erfindungsgemäßen Haarbehandlungsmittel, welches neben natürlichen oder synthetischen Polymeren Ilexharz enthält, behandelt wurden, zeichnen sich durch eine verbesserte Elastizität, mehr Glanz sowie überraschenderweise auch durch eine verbesserte Festigung aus.

Emulsionen, welche das erfindungsgemäße Ilexharz enthalten, weisen bei Temperaturen um 40° C eine größere Emulsionsstabilität gegenüber Emulsionen ohne einen Gehalt an Ilexharz auf.

Die folgenden Beispiele sollen den Gegenstand der Erfindung näher erläutern.

**Beispiele**

**Beispiel 1: Gewinnung von Ilexharz**

1 kg Matetee (Ilex paraguariensis) oder 1 kg Stechpalmenblätter (Ilex aquifolium) werden mit 3,2 l Essigsäureethylester 2 Stunden lang unter Rückfluß extrahiert. Anschließend wird bei einer Temperatur von mindestens 60° Celsius heiß filtriert. Der Rückstand wird mit 2,8 1 Essigsäureethylester versetzt und 1 Stunde unter Rückfluß gerührt. Anschließend wird heiß filtriert. Die Nachextraktion kann mehrmals wiederholt werden. Die Ausbeute nimmt dabei um jeweils etwa 0,3 % pro Nachextraktionsschritt zu.

Um Chlorophyll, Triterpene, Coffein und Glykoside zu entfernen, werden die vereinigten Filtrate aus den einzelnen Extraktionen zur Trockne eingeengt. Der dunkelgrüne Rückstand wird in ca. 1 l Hexan oder Petroläther (Siedebereich 60 - 80° Celsius) aufgenommen und erwärmt. Es bleibt dabei ein Rest aus polaren Komponenten ungelöst. Unter Rühren werden, noch vor Erreichen der Siedetemperatur, 35 g Bleicherde (Tonsil Supreme FF der Firma Süd-Chemie, München/Deutschland) zugegeben. Nun wird weiter erwärmt und noch weitere 15 min. unter Rückfluß gerührt. Anschließend wird der Bleicherde noch etwa 10 min. Zeit gegeben, sich abzusetzen. Dann wird heiß filtriert. Das Filtrat wird zur Trockne eingeengt (Vakuum ≤ 100 mbar, Wasserbad ca. 70° Celsius).

**Ausbeute**: ca. 24 g orange-gelb gefärbtes, durchscheinendes, zähes Ilexharz.

Eine Analyse des Ilexharzes ergibt, daß es gesättigte und ungesättigte, langkettige Kohlenwasserstoffe, freie Fettsäuren und gesättigte und ungesättigte Fettsäureester enthält. Chromatogramme und physikochemische Eigenschaften weisen auf eine weitgehende Übereinstimmung des aus Matetee und des aus Stechpalmenblättern gewonnenen Harzes hin.

**Beispiel 2: Hautcreme gegen auslaugende Einflüsse**

| | |
|---|---|
| 10,0 g | Ilexharz gemäß Beispiel 1 |
| 10,0 g | Paraffinum perliquidum |
| 15,0 g | Lanolinalkoholextrakt (Amerchol L101 der Firma Amerchol, Edison NJ, USA) |
| 10,0 g | Lanolin, anhydr. |
| 8,0 g | Glycerinmonodistearat |
| 0,3 g | p-Hydroxybenzoesäuremethylester / p-Hydroxybenzoesäurepropylester 70:30 |
| 0,2 g | Parfümöl |

(fortgesetzt)

| 46,5 g | Wasser |
|---|---|
| 100,0 g | |

**Beispiel 3: Hautschutzcreme (w/o-Emulsion)**

| | A | B | C |
|---|---|---|---|
| Ilexharz gem. Beispiel 1 | 10,0 g | 0,10 g | 20,0 g |
| Lanolinalkohol-extrakt (Amerchol L101 der Firma Amerchol, Edison NJ, USA) | 15,0 g | 15,00 g | 15,0 g |
| Paraffinum perliquidum | 10,0 g | 19,90 g | - |
| Lanolin anhydr. | 10,0 g | 10,00 g | 10,0 g |
| Glycerinmono-distearat | 8,0 g | 8,00 g | 8,0 g |
| Wasser | 47,0 g | 47,00 g | 47,0 g |
| | 100,0 g | 100,00 g | 100,0 g |

|  | D | E |
|---|---|---|
| Ilexharz gem. Bispiel 1 | 40,0 g | 50,0 g |
| Lanolinalkohol-extrakt (Amerchol L101 der Firma Amerchol, Edison NJ, USA) | 15,0 g | 15,0 g |
| Paraffinum perliquidum | - | - |
| Lanolin anhydr. | 10,0 g | 10,0 g |
| Glycerinmono-distearat | 8,0 g | 8,0 g |
| Wasser | 27,0 g | 17,0 g |
|  | 100,0 g | 100,0 g |

Der pH-Wert der Emulsion liegt zwischen pH 6 und 7.

**Beispiel 4: Handcreme (o/w-Emulsion)**

| | A | B | C |
|---|---|---|---|
| Ilexharz gem. Beispiel 1 | 10,0 g | 0,10 g | 20,0 g |
| Glycerin (86prozentig) | 15,0 g | 15,00 g | 15,0 g |
| Paraffinum subliquidum | - | 9,90 g | - |
| Cetyl/Stearyl-isononanoat | 5,0 g | 5,00 g | 5,0 g |
| Glycerinmono-distearat | 4,0 g | 4,00 g | 4,0 g |
| Wasser | 66,0 g | 66,00 g | 56,0 g |
| | 100,0 g | 100,00 g | 100,0 g |

| | D | E |
|---|---|---|
| Ilexharz gem. Beispiel 1 | 40,0 g | 50,0 g |
| Glycerin (86prozentig) | 15,0 g | 15,0 g |
| Paraffinum subliquidum | - | - |
| Cetyl/Stearyl-isononanoat | 5,0 g | 5,0 g |
| Glycerinmono-distearat | 4,0 g | 4,0 g |
| Wasser | 36,0 g | 26,0 g |
| | 100,0 g | 100,0 g |

Der pH-Wert der Handcreme liegt zwischen pH 6 und 7.

**Beispiel 5: Hautschutzgel**

|  | A | B | C | D |
|---|---|---|---|---|
| Ilexharz gem. Beispiel 1 | 0,10 g | 0,50 g | 1,0 g | 10,0 g |
| Glycerin (86prozentig) | 30,00 g | 30,00 g | 30,0 g | 30,0 g |
| Celluloseether | 4,00 g | 4,00 g | 4,0 g | 4,0 g |
| Hydr. Rizinusöl, ethoxyliert mit 45 mol Ethylenoxid | 0,20 g | 1,00 g | 2,0 g | 20,0 g |
| Wasser | 65,70 g | 64,50 g | 63,0 g | 36,0 g |
|  | 100,00 g | 100,00 g | 100,0 g | 100,0 g |

Der pH-Wert des Hautschutzgels liegt zwischen pH 5 und 6.

**Beispiel 6: Mikroemulsion zur Verbesserung von Glanz und Farbtiefe von Haaren**

| | |
|---|---|
| 7,25 g | Ilexharz gemäß Beispiel 1 |
| 10,00 g | Paraffinum perliquidum |
| 9,00 g | Tetraoxyethylenlaurylether |
| 3,75 g | Dioxyethylenlaurylether |
| 1,75 g | Cetyltrimethylammoniumchlorid |
| 0,20 g | Parfümöl |
| 0,20 g | Zitronensäure |
| 0,15 g | Sorbinsäure |
| 67,80 g | Wasser |
| 100,00 | |

**Beispiel 7: Klare Glanzcreme für das Haar**

| | A | B | C | D |
|---|---|---|---|---|
| Ilexharz gem. Beispiel 1 | 0,25 g | 1,00 g | 2,00 g | 3,00 g |
| Paraffinum perliquidum | 17,00 g | 16,25 g | 15,25 g | 14,25 g |
| Tetraoxyethy-lenlaurylether | 9,00 g | 9,00 g | 9,00 g | 9,00 g |
| Dioxyethylen-laurylether | 3,75 g | 3,75 g | 3,75 g | 3,75 g |
| Cetyltrimethyl-ammoniumchlorid | 1,75 g | 1,75 g | 1,75 g | 1,75 g |
| Zitronensäure | 0,20 g | 0,20 g | 0,20 g | 0,20 g |
| Sorbinsäure | 0,15 g | 0,15 g | 0,15 g | 0,15 g |
| Wasser | 67,90 g | 67,90 g | 67,90 g | 67,90 g |
| | 100,00 g | 100,00 g | 100,0 g | 100,0 g |

|  | E | F | G |
|---|---|---|---|
| Ilexharz gem. Beispiel 1 | 4,00 g | 5,00 g | 7,25 g |
| Paraffinum perliquidum | 13,25 g | 12,25 g | 10,00 g |
| Tetraoxyethy-lenlaurylether | 9,00 g | 9,00 g | 9,00 g |
| Dioxyethylen-laurylether | 3,75 g | 3,75 g | 3,75 g |
| Cetyltrimethyl-ammoniumchlorid | 1,75 g | 1,75 g | 1,75 g |
| Zitronensäure | 0,20 g | 0,20 g | 0,20 g |
| Sorbinsäure | 0,15 g | 0,15 g | 0,15 g |
| Wasser | 67,90 g | 67,90 g | 67,90 g |
|  | 100,00 g | 100,00 g | 100,00 g |

Der pH-Wert der Glanzcreme liegt zwischen pH 3 und 4.

**Beispiel 8: Schaumfestiger**

| | |
|---|---|
| 0,1 g | Ilexharz gemäß Beispiel 1 |
| 10,0 g | Ethanol |
| 8,0 g | Propan/Butan 5,0 bar |
| 0,8 g | Chitosan |
| 0,2 g | Ameisensäure |
| 0,2 g | Methylvinylimidazoliumchlorid/Vinylpyrrolidon Copolymer |
| 0,2 g | hydriertes Rizinusöl, ethoxyliert mit 40 Mol Ethylenoxid |
| 0,2 g | Kräuterextrakt (Extrapone 5 Spezial der Firma Dragoco, Deutschland) |
| 0,1 g | Tetraoxyethylenlaurylether |
| 0,1 g | Parfüm |
| 80,1 g | Wasser |
| 100,0 g | |

**Beispiel 9: Haarspray**

| | |
|---|---|
| 0,5 g | Ilexharz gemäß Beispiel 1 |
| 2,5 g | Acrylate/Acrylamid Copolymer |

(fortgesetzt)

| | |
|---|---|
| 0,2 g | 2-Amino-2-methyl-propanol |
| 0,1 g | Parfüm |
| 46,7 g | Ethanol |
| 50,0 g | Butan 1,5 bar |
| 100,0 g | |

**Beispiel 10: Pumpspray**

| | |
|---|---|
| 0,34 g | Ilexharz gemäß Beispiel 1 |
| 10,00 g | Vinylpyrrolidon/Vinylacetat Copolymer, 50 % in Ethanol |
| 0,30 g | Schellack |
| 0,15 g | Parfüm |
| 0,01 g | 2-Amino-2-methyl-propanol |
| 5,00 g | Wasser |
| 84,20 g | Ethanol |
| 100,00 g | |

**Beispiel 11: Flüssigfestiger mit Elastizitätskomponente**

| | |
|---|---|
| 0,5 g | Ilexharz gemäß Beispiel 1 |
| 7,0 g | Isodekan |
| 2,5 g | Vinylacetat/Crotonsäure Copolymer |
| 0,2 g | Parfüm |
| 0,1 g | Cetyltrimethylammoniumbromid |
| 0,1 g | Glycerin |
| 44,6 g | Wasser |
| 45,0 g | Ethanol |
| 100,0 g | |

**Beispiel 12: Haarreinigungsmittel mit konditionierender Wirkung**

| | |
|---|---|
| 0,6 g | Ilexharz gemäß Beispiel 1 |
| 9,6 g | Natriumlaurylethersulfat |
| 3,5 g | Natriumchlorid |
| 2,0 g | Polyethylenglykol-(3)-Distearat |
| 1,5 g | Kokosfettsäureamidopropylbetain |
| 0,4 g | Parfümöl |
| 0,3 g | Vinylpyrrolidon/Dimethylaminoethylmethacrylat Copolymer |
| 82,1 g | Wasser |
| 100,0 g | |

**Beispiel 13: auszuspülendes Haarpflegemittel**

| | |
|---|---|
| 1,5 g | Ilexharz gemäß Beispiel 1 |
| 2,5 g | Cetylstearylalkohol |
| 0,4 g | Cetyltrimethylammoniumchlorid |
| 0,4 g | Parfümöl |
| 0,3 g | Zitronensäure |
| 94,9 g | Wasser |
| 100,0 g | |

**Beispiel 14: nicht auszuspülendes Haarpflegemittel**

| | |
|---|---|
| 0,7 g | Ilexharz gemäß Beispiel 1 |
| 0,6 g | Cetylstearylalkohol |
| 0,4 g | Vinylpyrrolidon/Dimethylaminoethylmethacrylat Copolymer |
| 0,2 g | Cetyltrimethylammoniumchlorid |
| 0,2 g | Zitronensäure |
| 0,2 g | Parfümöl |
| 97,7 g | Wasser |
| 100,0 g | |

**Beispiel 15: versprühbares, nicht auszuspülendes Haarpflegemittel**

| | |
|---|---|
| 0,20 g | Ilexharz gemäß Beispiel 1 |
| 8,00 g | Ethanol |
| 0,45 g | Hydriertes Tallowtrimethylammoniumchlorid |
| 0,20 g | Gemisch aus Glycerylstearat und Cetylstearylpolyethylenglykol-(20) (Teginacid® der Firma Th. Goldschmidt, Deutschland) |
| 0,15 g | Cetylstearylalkohol |
| 0,13 g | N-Stearylbetain |
| 0,10 g | Parfümöl |
| 90,77 g | Wasser |
| 100,00 g | |

**Beispiel 16: auszuspülender Haarpflegeschaum**

| | |
|---|---|
| 1,1 g | Ilexharz gemäß Beispiel 1 |
| 3,0 g | Cetylstearylalkohol |
| 1,2 g | Laurylpolyglucose, 50%ig (Plantaren® 1200 CS/UP der Firma Henkel KGaA, Deutschland) |
| 1,2 g | Cetyltrimethylammoniumchlorid |
| 0,4 g | Parfümöl |
| 93,1 g | Wasser |
| 100,0 g | |

94,0 g des vorstehenden Haarpflegemittels werden mit 6 g eines Gemisches aus 75 Gew. % n-Butan, 20 Gew. % i-Butan und 5 Gew. % Propan in einem geeigneten Aerosolbehälter abgefüllt. Das erfindungsgemäße Haarpflegemittel wird dem Behälter in Form eines Schaumes entnommen.

**Beispiel 17: im Haar verbleibender Haarpflegeschaum**

| | |
|---|---|
| 0,7 g | Ilexharz gemäß Beispiel 1 |
| 4,5 g | Isododekan |
| 1,0 g | diquaternäres Polydimethylsiloxan (Abil® Quat 3272 der Firma Th. Goldschmidt, Deutschland) |
| 0,5 g | Polyvinylpyrrolidon |
| 0,4 g | α-Hydro-ω-hydroxypolyoxydimethylsilylen (CTFA: Dimethiconol) 13%ig in cyclischem Dimethylpolysiloxan (CTFA: Cyclomethicone) (Dow Corning Q2 1401 Fluid der Firma Dow Corning Europe, Belgien) |
| 0,2 g | Cetyltrimethylammoniumchlorid |
| 0,2 g | Parfümöl |
| 0,1 g | Vinylpyrrolidon/Dimethylaminoethylmethacrylat Copolymer |
| 97,4 g | Wasser |
| 100,0 g | |

96 g des vorstehenden Haarpflegemittels werden mit 4 g eines Gemisches aus 50 Gew. % Propan, 40 Gew. % n-Butan und 10 Gew. % Dimethylether in einen Aerosolbehälter abgefüllt. Das erfindungsgemäße Haarpflegemittel wird beim Versprühen in Schaumform dem Behälter entnommen.

**Beispiel 18: auszuspülendes Haarpflegemittel**

| | |
|---|---|
| 1,40 g | Ilexharz gemäß Beispiel 1 |
| 5,20 g | Cetylstearylalkohol |
| 2,70 g | Wollfett |
| 2,00 g | Glycerinmonodistearat mit Kaliumdistearat |
| 1,17 g | Natriumcetylstearylsulfat |
| 0,80 g | Wollwachs |
| 0,50 g | Gemisch aus Lanolin und Lanolinalkohol (Aquaphil® K, vertrieben durch die Firma Deutsche Lanolin Gesellschaft, Deutschland) |
| 0,30 g | Parfümöl |
| 0,20 g | Cholesterin |
| 85,73 g | Wasser |
| 100,00 g | |

**Beispiel 19: Vergleichsversuch zur Hautschutzwirkung**

| | |
|---|---|
| 20,0 g | Paraffinum perliquidum |
| 15,0 g | Lanolinalkoholextrakt (Amerchol L101 der Firma Amerchol, Edison NJ, USA) |
| 10,0 g | Lanolin anhydr. |
| 8,0 g | Glycerinmonodistearat |
| 0,3 g | p-Hydroxybenzoesäuremethylester/p-Hydroxybenzoesäurepropylester 70:30 |
| 0,2 g | Parfümöl |
| 46,5 g | Wasser |
| 100,0 g | |

Die wasserabweisende Wirkung auf der Haut und die Waschbeständigkeit ist für die erfindungsgemäße Hautcreme nach Beispiel 2 gegenüber der Vergleichscreme nach Beispiel 19 erhöht.

EP 0 822 803 B1

**Beispiel 20: Vergleichsversuch zur Haarpflegewirkung**

| | |
|---|---|
| 17,25 g | Paraffinum perliquidum |
| 9,00 g | Tetraoxyethylenlaurylether |
| 3,75 g | Dioxyethylenlaurylether |
| 1,75 g | Cetyltrimethylammoniumchlorid |
| 0,20 g | Parfümöl |
| 0,20 g | Zitronensäure |
| 0,15 g | Sorbinsäure |
| 67,80 g | Wasser |
| 100,00 g | |

Haar, welches mit der Mikroemulsion gemäß Beispiel 6 behandelt wurde, wies gegenüber mit der Emulsion gemäß Beispiel 20 behandeltem Haar mehr Glanz und eine größere Farbtiefe aus.

Sämtliche in dieser Anmeldung angegebenen Prozentzahlen stellen Gewichtsprozente dar.

**Patentansprüche**

1. Kosmetisches Mittel mit einem Gehalt an Ilexharz, welches aus Blättern von Ilex aquifolium oder Ilex paraguariensis isoliert wird.

2. Mittel nach Anspruch 1 zur Hautbehandlung, **dadurch gekennzeichnet, daß** es 0,001 bis 50 Gewichtsprozent Ilexharz enthält.

3. Mittel nach Anspruch 1 zur Haarbehandlung, **dadurch gekennzeichnet, daß** es 0,001 bis 10 Gewichtsprozent Ilexharz enthält.

4. Mittel nach Anspruch 1 zur Reinigung der Haare, **dadurch gekennzeichnet, daß** es zusätzlich 0,01 bis 40 Gew. % mindestens eines anionischen, amphoteren oder nichtionischen Tensids enthält.

5. Mittel nach Anspruch 4, **dadurch gekennzeichnet, daß** es zusätzlich 0,01 bis 8 Gew. % mindestens eines kationischen Tensids enthält.

6. Mittel nach Anspruch 1 zur Pflege der Haare, **dadurch gekennzeichnet, daß** es zusätzlich (a), 0,01 bis 30 Gew. % mindestens eines haarpflegenden Wirkstoffs, ausgewählt aus Lipiden, Paraffinölen und Silikonverbindungen sowie (b) 0,03 - 8 Gew. % Emulgatoren, ausgewählt aus kationischen, anionischen, amphoteren und nichtionischen Tensiden enthält.

7. Mittel nach Anspruch 1, **dadurch gekennzeichnet, daß** es zusätzlich mindestens ein natürliches oder synthetisches Polymer enthält.

8. Mittel nach Anspruch 7 zur Festigung der Haare, **dadurch gekennzeichnet, daß** es

   (A) 0,001 bis 10 Gewichtsprozent Ilexharz

   (B) 0,01 bis 25 Gewichtsprozent mindestens eines natürlichen oder synthetischen Polymeren

   (C) 30 bis 99,89 Gewichtsprozent eines geeigneten organischen, wäßrig-organischen oder wäßrigen Lösungsmittels

   enthält.

9. Verwendung von Ilexharz als Haarglanzverstärker.

17

**10.** Verwendung von Ilexharz zur Erhöhung der Lagerstabilität von o/w- oder w/o-Emulsionen.

**11.** Verfahren zur Gewinnung von Ilexharz aus den Blättern von Ilex aquifolium oder Ilex paraguariensis durch Extraktion mit einem semipolaren oder apolaren, wasserfreien organischen Lösungsmittel oder mit überkritischem Kohlendioxid.

**12.** Verfahren nach Anspruch 11, bei dem man (a) die Blätter mindestens zweimal mit Essigsäureethylester heiß extrahiert und heiß filtriert und das Filtrat zur Trockne einengt, (b) den Rückstand in einem unpolaren organischen Lösungsmittel aufnimmt, in der Wärme Bleicherde zugibt, zum Sieden erhitzt, heiß filtriert und das Filtrat zur Trockene einengt.

**13.** Ilexharz, welches aus Blättern von Ilex paraguariensis oder Ilex aquifolium nach dem Verfahren gemäß Anspruch 11 oder 12 isoliert wird.

## Claims

**1.** Cosmetic agent containing ilex resin which is isolated from leaves of <u>Ilex aquifolium</u> or <u>Ilex paraquariensis</u>.

**2.** Agent according to Claim 1 for skin treatment, **characterised in that** it contains from 0.001 to 50% by weight of ilex resin.

**3.** Agent according to Claim 1 for hair treatment, **characterised in that** it contains from 0.001 to 10% by weight of ilex resin.

**4.** Agent according to Claim 1 for cleaning hair, **characterised in that** it contains, in addition, from 0.01 to 40% by weight of at least one anionic, amphoteric or non-ionic surfactant.

**5.** Agent according to Claim 4, **characterised in that** it contains, in addition, from 0.01 to 8% by weight of at least one cationic surfactant.

**6.** Agent according to Claim 1 for hair care, **characterised in that** it contains, in addition, (a) from 0.01 to 30% by weight of at least one active hair care substance, selected from lipids, paraffin oils and silicone compounds and (b) from 0.03 to 8% by weight of emulsifiers, selected from cationic, anionic, amphoteric and non-ionic surfactants.

**7.** Agent according to Claim 1, **characterised in that** it contains, in addition, at least one natural or synthetic polymer.

**8.** Agent according to Claim 7 for setting the hair, **characterised in that** it contains:

(A) from 0.001 to 10% by weight of ilex resin,

(B) from 0.01 to 25% by weight of at least one natural or synthetic polymer,

(C) from 30 to 99.89% by weight of a suitable organic, aqueous-organic or aqueous solvent.

**9.** Use of ilex resin to enhance hair shine.

**10.** Use of ilex resin for increasing the stability of oil-in-water or water-in-oil emulsions during storage.

**11.** Process for obtaining ilex resin from the leaves of <u>Ilex aquifolium</u> or <u>Ilex paraquariensis</u> by extraction with a semipolar or non-polar, anhydrous, organic solvent or with supercritical carbon dioxide.

**12.** Process according to Claim 11, in which (a) the leaves are hot-extracted and hot-filtered at least twice with ethyl acetate and the filtrate is concentrated to dryness, (b) the residue is taken up in a non-polar, organic solvent, bleaching earth is added at elevated temperature, the whole is heated to boiling point and hot-filtered and the filtrate is concentrated to dryness.

**13.** Ilex resin which is isolated from leaves of <u>Ilex paraquariensis</u> or <u>Ilex aquifolium</u> by means of the process according to Claim 11 or 12.

**Revendications**

1.  Agent cosmétique présentant une teneur en résine d'ilex, ayant été isolé à partir de feuilles d'ilex aquifolium ou d'ilex paraguariensis.

2.  Agent selon la revendication 1 pour le traitement de la peau, **caractérisé en ce qu'**il contient de 0,001 à 50 pour-cent en poids de résine d'ilex.

3.  Agent selon la revendication 1 pour le traitement des cheveux, **caractérisé en ce qu'**il contient de 0,001 à 10 pour-cent en poids de résine d'ilex.

4.  Agent selon la revendication 1 pour le nettoyage de la peau, **caractérisé en ce qu'**il contient en plus de 0,01 à 40 pour-cent en poids d'au moins un tensioactif anionique, amphotère ou non ionique.

5.  Agent selon la revendication 4, **caractérisé en ce qu'**il contient en plus de 0,01 à 8 pour-cent en poids d'au moins un tensioactif cationique.

6.  Agent selon la revendication 1 pour les soins des cheveux, **caractérisé en ce qu'**il contient en plus (a) de 0,01 à 30 pour-cent en poids d'au moins un principe actif destiné au soin des cheveux, sélectionné parmi les lipides, les huiles de paraffine et les combinaisons silicone ainsi que (b) de 0,03 à 8 pour-cent en poids d'émulsifiants sélectionnés à partir de tensioactifs cationiques, anioniques, amphotères et non ioniques.

7.  Agent selon la revendication 1, **caractérisé en ce qu'**il contient en plus au moins un polymère naturel ou synthétique.

8.  Agent selon la revendication 7, pour la fixation des cheveux, **caractérisé en ce qu'**il contient

    (A) de 0,001 à 10 pour-cent en poids de résine d'ilex,

    (B) de 0,01 à 25 pour-cent en poids d'au moins un polymère naturel ou synthétique,

    (C) de 30 à 99,89 pour-cent en poids d'un solvant organique, organo-aqueux ou aqueux, approprié.

9.  Utilisation de résine d'ilex comme renforçateur du lustrant des cheveux.

10. Utilisation de résine d'ilex pour augmenter la stabilité au stockage d'émulsions aqueuses ou huileuses.

11. Procédé d'obtention de résine d'ilex à partir des feuilles d'ilex aquifolium ou d'ilex paraguariensis, par extraction avec un solvant organique non aqueux, semi-polaire ou apolaire, ou bien à l'aide de dioxyde de carbone surcritique.

12. Procédé selon la revendication 11, dans lequel (a) on procède à une extraction à partir des feuilles au moins à deux reprises, avec de l'éthylester d'acide acétique, et on filtre à chaud et on concentre le filtrat jusqu'à obtention d'un produit sec, (b) on place le dépôt dans un solvant organique non polaire, on ajoute de l'argile absorbante-décolorante en présence de chaleur, on chauffe jusqu'à ébullition, on filtre à chaud et on concentre le filtrat jusqu'à obtention d'un produit sec.

13. Résine d'ilex, ayant été isolée à partir de feuilles d'ilex paraguariensis ou d'ilex aquifolium suivant le procédé selon la revendication 11 ou 12.